(19) Europäisches Patentamt
European Patent Office
Office européen des brevets

(11) Veröffentlichungsnummer: **0 003 316 B1**

# (12) EUROPÄISCHE PATENTSCHRIFT

(45) Veröffentlichungstag der Patentschrift: **30.12.81**

(51) Int. Cl.³: **C 07 D 407/04**

(21) Anmeldenummer: **79100133.2**

(22) Anmeldetag: **17.01.79**

(54) **Stereospezifisches Verfahren für die Herstellung von 2-Oxiranyl-1,4-benzodioxanen.**

(30) Priorität: **30.01.78 US 873456**

(43) Veröffentlichungstag der Anmeldung: **08.08.79 Patentblatt 79/16**

(45) Bekanntmachung des Hinweises auf die Patenterteilung: **30.12.81 Patentblatt 81/52**

(84) Benannte Vertragsstaaten: **BE CH DE FR GB IT NL SE**

(56) Entgegenhaltungen: **Keine Entgegenhaltungen.**

(73) Patentinhaber: **CIBA-GEIGY AG, Patentabteilung Postfach, CH-4002 Basel (CH)**

(72) Erfinder: **Gschwend, Heinz Werner, Dr., 112 Sherwood Drive, New Providence New Jersey (US)**
Erfinder: **Huebner, Charles Ferdinand, Dr., 40 Edgewood Road, Chatham New Jersey (US)**

EP 0 003 316 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Stereospezifisches Verfahren für die Herstellung von 2-Oxiranyl-1,4-benzodioxanen

In den USA-Patenten Nrn 3101345; 3312592; 3910930 und 3914238 sind pharmakologisch wirksame 2-(2-Amino-1-hydroxyäthyl)-1,4-benzodioxane offenbart. Diese werden verfahrensgemäss als Gemische von Diastereoisomeren erhalten, welche man lediglich nach schwerfälligen Methoden auftrennen kann. Die vorliegende Erfindung stellt ein Verfahren bereit, welches Zwischenprodukte für die genannten Verbindungen in reinen erythro- und threo-Formen ergibt und lediglich einfache und billige Ausgangsstoffe benötigt. Überdies ist das neue Verfahren besonders geeignet für die einfache Herstellung von 1,4-Benzodioxanen, welche im o-Phenylenring substituiert sind. Diese Verbindungen sind wertvolle Ausgangsstoffe für die Herstellung von neuen erythro-2-(2-Aralkylamino-1-hydroxyäthyl)-1,4-benzodioxanen.

Die vorliegende Erfindung betrifft ein neues stereospezifisches Verfahren für die Herstellung von 2-Oxiranyl-1,4-benzodioxanen der allgemeinen Formel I

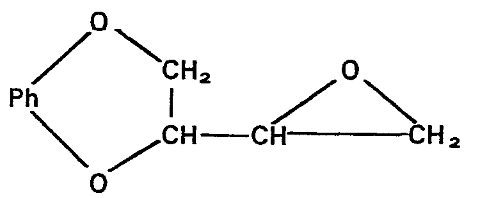

(I)

worin Ph 1,2-Phenylen bedeutet, welches durch ein bis drei gleiche oder verschiedene Substituenten der Gruppe bestehend aus Niederalkyl, Niederalkoxy, Niederalkylendioxy, Benzyloxy, Halogen, Trifluormethyl oder Nitro substituiert sein kann. Diese Verbindungen sind wertvolle Ausgangsstoffe für die Herstellung von therapeutisch nützlichen Produkten, z.B. solchen, welche β-adrenergisch blockierende oder antihypertensive Wirkungen aufweisen.

Die erfindungsgemäss erhaltenen Produkte sind d,l-erythro- oder d,l-threo-Verbindungen. Im erythro-Isomeren ist das Sauerstoffatom des Oxiranringes in Bezug auf das benachbarte Sauerstoffatom des Dioxanrings in relativer erythro-Orientierung. Im threo-Isomeren ist die relative Orientierung der beiden Sauerstoffatome threo.

Das stereospezifische Verfahren der Erfindung besteht darin, dass man in Gegenwart einer starken Base, eine Verbindung der allgemeinen Formel II

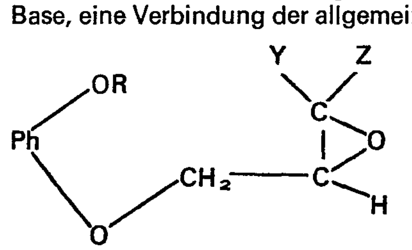

(II)

kondensiert, worin R Formyl oder M bedeutet, M für den Rest einer starken quaternären organischen Stickstoff enthaltenden Base oder für ein Alkalimetallatom $M_a$ steht, eines der Symbol Y und Z Wasserstoff bedeutet und das andere für $X-CH_2$ steht, worin X ein Halogenatom bedeutet, oder, wenn R für M steht, das Symbol X auch eine andere reaktionsfähige veresterte Hydroxygruppe bedeutet.

Die Umwandlung der gemäss der Erfindung erhaltenen Verbindungen der Formel I in therapeutisch wertvolle Produkte ist in den oben genannten Patenten offenbart, jedoch ohne Hinweis auf stereochemisch einheitliche Gruppen, von welchen überraschenderweise diejenigen mit erythro-Form fast die ganze pharmakologische Wirkung aufweisen.

Die Substituenten des Oxiranrings im Ausgangsstoff der Formel II können zueinander in cis-Konfiguration (Z bedeutet Wasserstoff) oder in trans-Konfiguration (Z steht für die Gruppe $CH_2X$) sein. Das zuerst genannte Isomere führt zu den threo-Verbindungen der Formel I, während die trans-Ausgangsstoffe erythro-Produkte der Formel I ergeben.

Die starke Base wird durch die Formel $M^{\oplus} OH^{\ominus}$ charakterisiert, worin $M^{\oplus}$ das vom M abgeleitete Kation ist. Das Symbol M ist insbesondere der Rest einer quaternären organischen Stickstoff enthaltenden Verbindung oder ein Alkalimetallatom, wie Lithium, Natrium oder vorzugsweise Kalium. Der Rest einer quaternären organischen Stickstoff enthaltenden Verbindung ist z.B. ein Tetraniederalkylammonium-, wie Tetraäthylammonium-, Tetrabutylammonium-, Trimethylbutylammonium-, aber auch ein Benzyltriniederalkylammonium-, z.B. Benzyltrimethylammoniumrest, oder eine analoge gesättigte heterocyclische Gruppe, z.B. ein N,N-Dimethylpiperidinium oder N,N-Dimethylpyrrolidinium- oder ein Aryltriniederalkylammonium-, z.B. ein Phenyltrimethylammoniumrest.

Eine reaktionsfähige veresterte Hydroxygruppe X ist vorzugsweise ein Halogenatom, insbesondere Chlor, aber auch Brom oder Jod; oder eine aliphatische oder aromatische Sulfonyloxygruppe, z.B. Methansulfonyloxy-, Benzolsulfonyloxy-, Toluolsulfonyloxy- oder Brombenzolsulfonyloxygruppe.

Der 1,2-Phenylenrest Ph ist vorzugsweise unsubstituiert oder monosubstituiert. Seine Substituenten sind durch die folgenden Gruppen illustriert: Niederalkyl, z.B. Methyl, Äthyl, n- oder Isopropyl oder -Butyl; Niederalkoxy, z.B. Methoxy, Äthoxy, n- oder Isopropoxy oder -Butoxy; Niederalkylendioxy, z.B. Methylendioxy, 1,1- oder 1,2-Äthylendioxy; Benzyloxy; Halogen, z.B. Fluor, Chlor oder Brom, Trifluormethyl oder Nitro.

Bevorzugte Verbindungen sind diejenigen der Formel I, worin Ph 1,2-Phenylen bedeutet, welches durch Alkyl oder Alkoxy mit jeweils 1-4 Kohlenstoffatomen, Alkylendioxy mit 1 oder 2 Kohlenstoffatomen, Benzyloxy, Fluor, Chlor, Brom, Trifluormethyl oder Nitro monosubstituiert sein kann.

Besonders hervorzuheben sind Verbindungen der Formel I, worin Ph für 1,2-Phenylen steht, welches durch Methyl, Methoxy, Methylendioxy, Benzyloxy, Fluor, Chlor, Trifluormethyl oder Nitro monosubstituiert sein kann.

Die Ausgangsstoffe der Formel II können gemäss den folgenden Verfahrensschritten hergestellt werden:

1) 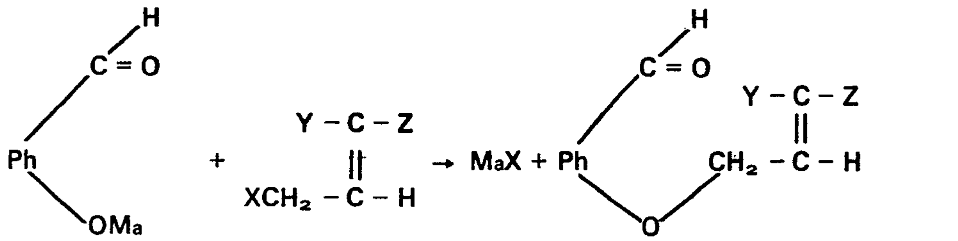 (III)

worin $M_a$ ein Alkalimetallatom bedeutet, X für ein Halogenatom steht, eines der Symbole Y und Z Wasserstoff bedeutet und das andere für $CH_2X$ steht;

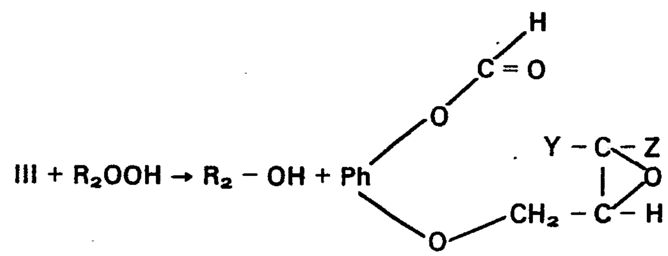 (II)

worin $R_2$ Niederalkanoyl, Halogenniederalkanoyl, HPhCO, $HOOC-C_2H_4-CO$ oder $HOOC-Ph-CO$ bedeutet.

Wenn Y in einem Ausgangsstoff der Formel II Wasserstoff bedeutet (welcher Ausgangsstoff ausgehend vom trans-Olefin erhalten worden ist), so werden erythro-Produkte der Formel I erhalten. Geht man von Verbindungen der Formel II aus, in welchen das Symbol Z für Wasserstoff steht (welche Verbindungen von cis-Olefinen stammen), so erhält man threo-Verbindungen als Produkte der Formel I.

Im Verfahrensschritt 2) verbraucht sowohl die Baeyer-Villiger-Reaktion als auch die Epoxydation ein Moläquivalent einer der oben genannten Persäuren, z.B. Peressig-, Pertrifluoressig-, Perbenzoe-, 3-chlorperbenzoe-, Perbernstein- oder Monoperphthalsäure. Je nach der Verwendung von cis- oder trans-Olefinen ist der stereospezifische Verlauf des Verfahrens gewährleistet.

Die Ausgangsstoffe der Reaktion 1) sind bekannt oder, wenn neu, können sie gemäss den an sich bekannten Methoden hergestellt werden.

Die oben genannten Reaktionsschritte werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt. So werden z.B. der Kondensationsschritt 1) und die Kondensation gemäss der Reaktion der vorliegenden Erfindung in einem wässerigen Medium, welches Phasentransferkatalysatoren, z.B. Tetraniederalkylammoniumhydroxyde, z.B. Tetrabutylammoniumhydroxyd, oder Kronenäther, und/oder Niederalkanolen, z.B. Methanol, Äthanol oder Isopropanol durchgeführt.

Die Peroxydation wird in niedrig siedenden Halogenalkanen, z.B. Methylenchlorid für die genannten aromatischen Persäuren; Niederalkylalkanoaten, z.B. Essigsäureäthylester für aliphatische Persäuren; Dimethylformamid für Succinoylperoxyd, vorgenommen. Ein Alkalimetallhydrogencarbonat, z.B. Kaliumhydrogencarbonat oder -carbonat kann mit Pertrifluoressigsäure verwendet werden, welches die überschüssige Persäure, nach dem Verbrauch des Olefins, zerstört.

So können die Ausgangsstoffe der Formel II unter den Reaktionsbedingungen des Hauptverfahrens gemäss dem folgenden Verfahrensschritt 3) gebildet werden:

3) 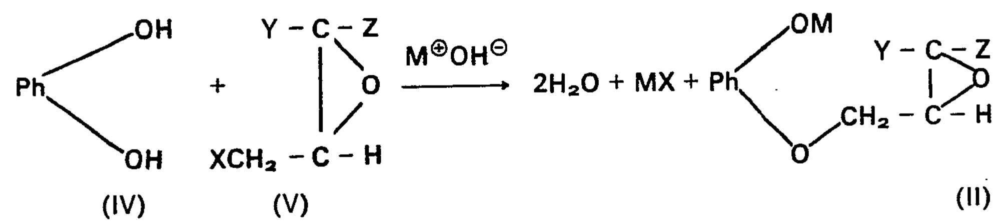


(IV) (V) (II)

worin alle Symbol die oben angegebenen Bedeutungen haben.

Das Oxiran-Reagens V ist entweder in cis- oder in trans-Form. Die erstgenannte Verbindung ergibt in der Reaktion des Hauptverfahrens threo-Verbindungen der Formel I, während aus der trans-Form erythro-Verfahrensprodukte entstehen. Bevorzugte Verbindungen der Formel V sind die trans-Isomeren, d.h. solche, in welchen Y Wasserstoff bedeutet und Z für die Gruppe $CH_2X$ steht.

In der Reaktion des Verfahrensschrittes 3) werden vorzugsweise solche Ausgangsstoffe verwendet, in welchen M Natrium bedeutet und X für ein Chloratom steht.

Die Reaktion 3) ist insbesondere für die in situ Vorbereitung von solchen Ausgangsstoffen geeignet, in welchen der 1,2-Phenylenring unsubstituiert oder symmetrisch substituiert ist. Solche sind Verbindungen der Formel II, worin Ph 1,2-Phenylen oder z.B. 3,6- oder 4,5-Dimethoxy-1,2-phenylen bedeutet.

Die Kondensation der Reaktion 3) wird, wie oben beschrieben, in einem wässerigen Medium, welches vorzugsweise ein Phasentransferkatalysator, z.B. Tetraniederalkylammoniumhydroxyd, z.B. Tetrabutylammoniumhydroxyd, oder Kronenäther enthält, durchgeführt. Sie kann auch in Anwesenheit von Verdünnungsmitteln, vorzugsweise in solchen, welche gegenüber den Reagenzien inert sind und diese lösen, z.B. in Niederalkylformamiden oder -sulfoxiden, wie Dimethylformamid oder -sulfoxid, von Katalysatoren, Kondensationsmitteln und/oder inerten Atmosphären, unter Kühlung, bei Zimmertemperatur oder bei erhöhten Temperaturen, vorzugsweise zwischen ungefähr 50°C und ungefähr 70°C, bei normalem oder erhöhtem Druck durchgeführt.

Die Ausgangsstoffe der Formeln IV und V sind bekannt oder, wenn neu, können sie gemäss den an sich bekannten Methoden hergestellt werden. So können z.B. die Salze von Verbindungen der Formel IV, ausgehend von entsprechenden Catechinen und starken Basen, wie Alkalimetallhydroxiden, -niederalkoxiden oder -hydriden oder Triniederalkylbenzylammoniumhydroxiden hergestellt werden.

Die Oxirane der Formel V können nach konventionellen Methoden, ausgehend von entsprechenden cis- oder trans-Butadiendihalogeniden und Epoxydation mit aliphatischen oder vorzugsweise aromatischen Persäuren, z.B. Peressig-, Perbernstein-, Perbenzoe-, m-Chlorperbenzoe- oder Monoperphthalsäure hergestellt werden. Die überschüssige Persäure kann, nach der Oxydation des Olefins, z.B. mit Natriumsulfit zerstört werden. Die genannten Oxirane können auch durch katalytische Oxydation mit Luft oder Sauerstoff in der Dampfphase, z.B. mit Silber-, Platin- oder Palladiumkatalysatoren, erhalten werden.

Im Verfahren der vorliegenden Anmeldung werden vorteilhafterweise solche Ausgangsstoffe verwendet, welche zu den im vorstehenden als besonders wertvoll beschriebenen Verbindungen, z.B. zu den in den obigen Patenten genannten Produkten, führen.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 15 und 100 mmHg, durchgeführt.

*Beispiel 1:*

Man lässt 1200 ml Wasser unter Rückfluss kochen und versetzt es unter kräftigen Rühren mit 1 ml 40%igem wässerigem Tetrabutylammoniumhydroxyd, 125 ml Salicylaldehyd und 150 ml cis-1,4-Dichlor-2-buten. Das Reaktionsgemisch wird dann durch tropfenweise Zugabe einer Lösung von 42 g Natriumhydroxyd in 200 ml Wasser bei einem pH-Wert zwischen 7 und 8 gehalten, unter Rückfluss bis zum pH-Wert 7 gekocht, abgekühlt und mit Methylenchlorid extrahiert. Der Extrakt wird mit 5%iger wässeriger Natriumhydroxydlösung gewaschen, getrocknet und eingedampft. Der Rückstand wird destilliert und die bei 137 bis 140°C/0,1 mmHg siedende Fraktion aufgefangen. Man erhält den 2-(4-Chlor-cis-2-butenyloxy)-benzaldehyd.

Eine Lösung von 24 g der letztgenannten Verbindung in 500 ml Methylenchlorid wird mit 0,2 g 2,4,6-Tributylphenol und 55 g 85%iger 3-Chlorperbenzoesäure versetzt und das Gemisch 3 Tage unter Rückfluss gekocht. Das Reaktionsgemisch wird abgekühlt, filtriert, das Filtrat mit 5%iger wässeriger Natriumsulfitlösung und 5%iger wässeriger Natriumhydrogencarbonatlösung gewaschen, getrocknet und eingedampft. Man erhält das

2-(4-Chlor-cis-2,3-epoxybutyloxy)phenol-formiat,

welches im NMR-Spektrum «Peaks» bei 3,4, 3,6, 4,2, 6,9 und 8,25 ppm aufweist.

Eine Lösung von 4,6 g der letztgenannten Verbindung in 24 ml Methanol wird in einer Stickstoffatmosphäre unter Rühren mit 24 ml einer 10%igen wässerigen Kaliumhydroxydlösung tropfenweise versetzt, wobei man die Temperatur unter 30°C hält. Das Reaktionsgemisch wird 18 Stunden bei Zimmertemperatur gerührt, dann zum Teil eingedampft, der Rückstand in Wasser aufgenommen, und das Gemisch mit Diäthyläther extrahiert. Der Extrakt wird mit Wasser gewaschen, getrocknet und eingedampft. Der Rückstand wird destilliert und die bei 120°C/0,2 mmHg siedende Fraktion aufgefangen. Man erhält das d,l-threo-2-Oxiranyl-1,4-benzodioxan der Formel

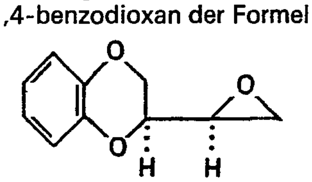

welches bei 45°C schmilzt.

*Beispiel 2:*

Ersetzt man im Beispiel 1 das cis-1,4-Dichlor-2-buten durch die gleiche Menge trans-1,4-Dichlor-2-buten und arbeitet man gemäss dem genannten Beispiel, so erhält man den 2-(4-Chlor-trans-2-butenyloxy)benzaldehyd, welcher bei 147 bis 148°C/0,15 mmHg siedet. (Diese Verbindung ist im deutschen Patent 1926023 genannt worden.)

Die Oxydation gemäss Beispiel 1 von 24 g der letztgenannten Verbindung ergibt das 2-(4-Chlor-trans-2,3-epoxybutyloxy)phenol-formiat, welches NMR-Spektrum «Peaks» bei 3,2, 3,5, 4,1, 7,0 und 8,2 ppm aufweist.

Gemäss Beispiel 1 werden 4,6 g der letztgenannten Verbindung ringgeschlossen. Man erhält das d,l-erythro-2-Oxiranyl-1,4-benzodioxan der Formel

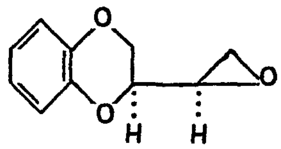

welches bei 120°C/0,2 mmHg siedet. Das Produkt kristallisiert und schmilzt bei 51 bis 52°C.

*Beispiel 3:*

Man lässt 2000 ml Wasser unter Rückfluss kochen und versetzt es unter kräftigem Rühren mit 3 ml 40%igem wässerigem Tetrabutylammoniumhydroxyd, 250 ml Salicylaldehyd und 280 ml trans-1,4-Dichlor-2-buten. Das Reaktionsgemisch wird dann durch tropfenweise Zugabe einer Lösung von 83 g Natriumhydroxyd in 400 ml Wasser bei einem pH-Wert zwischen 7 und 8 gehalten (ungefähr 25 Minuten). Nachher wird das Reaktionsgemisch ungefähr 6 Minuten, bis der pH-Wert ungefähr 7 ist, unter Rückfluss gekocht, auf 20°C gekühlt und die wässerige Schicht mit Diäthyläther extrahiert. Der Extrakt wird mit der organischen Schicht vereinigt, mit 5%iger wässeriger Natriumhydroxydlösung gewaschen, getrocknet und eingedampft. Der Rückstand wird destilliert und die bei 141 bis 147°C/0,7 mmHg siedende Fraktion aufgefangen. Man erhält den 2-(4-Chlor-trans-2-butenyloxy)benzaldehyd.

Eine Lösung von 155,7 g der letztgenannten Verbindung in 3800 ml Methylenchlorid wird mit 357 g 85%iger 3-Chlorperbenzoesäure versetzt, das Gemisch eine Stunde gerührt und 4 Tage unter Rückfluss gekocht. Das Reaktionsgemisch wird gekühlt, filtriert, das Filtrat mit Methylenchlorid und gesättigter wässeriger Natriumhydrogencarbonatlösung gewaschen, getrocknet und unter 40°C eingedampft. Man erhält das 2-(4-Chlor-trans-2,3-epoxybutyloxy)phenol-formiat, welches im NMR-Spektrum «Peaks» bei 3,2, 3,5, 4,1, 7,0 und 8,2 ppm zeigt.

Eine Lösung von 229,4 g der letztgenannten Verbindung in 900 ml Methanol wird unter Rühren in einer Stickstoffatmosphäre tropfenweise mit 1100 ml einer 10%igen wässerigen Kaliumhydroxydlösung versetzt, wobei man die Temperatur zwischen 8 und 15°C hält. Das Reaktionsgemisch wird über Nacht bei Zimmertemperatur gerührt, dann vom erhaltenen Niederschlag dekantiert und die Flüssigkeit mit Diäthyläther extrahiert. Der Niederschlag und der Extrakt werden vereinigt und das Gemisch wird mit Wasser gewaschen, getrocknet und eingedampft. Der Rückstand wird aus Diäthyläther umkristallisiert. Man erhält das d,l-erythro-2-Oxiranyl-1,4-benzodioxan, welches bei 51 bis 52°C schmilzt. Das Produkt ist mit demjenigen des Beispiels 2 identisch.

*Beispiel 4:*

Man lässt 1200 ml Wasser unter Rückfluss kochen und versetzt es unter kräftigem Rühren mit 1 ml 40%igem wässerigem Tetrabutylammoniumhydroxyd, 160 g 5-Chlorsalicylaldehyd und 140 ml trans-1,4-Dichlor-2-buten. Das Reaktionsgemisch wird dann durch tropfenweise Zugabe einer Lösung von 42 g Natriumhydroxyd in 200 ml Wasser bei einem pH-Wert zwischen 7 und 8 gehalten, unter Rückfluss bis zum pH-Wert 7 gekocht, abgekühlt und mit Methylenchlorid extrahiert. Der Extrakt wird mit 5%iger wässeriger Natriumhydroxydlösung gewaschen, getrocknet und eingedampft. Zum Abdampfen des überschüssigen 1,4-Dichlor-2-butens wird der Rückstand bei 0,1 mmHg auf 30°C erwärmt. Der Rückstand wird in 1000 ml Diäthyläther aufgenommen, die Lösung filtriert, das Filtrat eingedampft und der Rückstand aus Essigsäureäthylester umkristallisiert. Man erhält den 5-Chlor-2-(4-chlor-trans-2-butenyloxy)benzaldehyd, der bei 62 bis 63°C schmilzt.

Ein Gemisch von 6 g der letztgenannten Verbindung, 20 g 3-Chlorperbenzoesäure und 200 ml Methylenchlorid wird 3 Tage unter Rückfluss gekocht. Das Reaktionsgemisch wird abgekühlt, filtriert und das Filtrat mit 5%iger wässeriger Natriumsulfitlösung und mit 5%iger wässeriger Natriumhydrogencarbonatlösung gewaschen, getrocknet und eingedampft. Man erhält das 5-Chlor-2-(4-chlor-trans-2,3-epoxybutyloxy)-phenolformiat, welches im NMR-Spektrum «Peaks» bei 3,3, 3,6, 4,2 und 8,2 ppm aufweist.

Eine Lösung von 7 g der letztgenannten Verbindung in 25 ml Methanol wird unter Kühlen mit 30 ml einer 1,75-normalen wässerigen Kaliumhydroxydlösung versetzt, das Gemisch bei Zimmertemperatur 18 Stunden gerührt, mit Wasser verdünnt und mit Diäthyläther extrahiert. Der Extrakt wird mit Wasser gewaschen, getrocknet und eingedampft. Man erhält das d,l-erythro-6-Chlor-2-oxiranyl-1,4-benzodioxan als ein Öl, welches im NMR-Spektrum «Peaks» bei 2,8, 3,0 und 6,4 ppm aufweist und bei einer Kugelrohrdestillation bei 130°C/0,2 mmHg siedet.

*Beispiel 5:*

Ersetzt man den 4-Chlorsalicylaldehyd durch 152 g o-Vanillin und geht man genau gemäss Beispiel 4 vor, so wird der 2-(4-Chlor-trans-2-butenyloxy)-3-methoxybenzaldehyd erhalten, welcher bei 165°C/0,15 mmHg siedet.

Ein Gemisch von 7 g der letztgenannten Verbindung, 21 g 3-Chlorperbenzoesäure und 250 ml Methylenchlorid wird 3 Tage unter Rückfluss gekocht. Das Reaktionsgemisch wird abgekühlt, filtriert und das Filtrat mit 5%iger wässeriger Natriumsulfitlösung und mit 5%iger wässeriger Natriumhydrogencarbonatlösung gewaschen,

getrocknet und eingedampft. Man erhält das 2-(4-Chlor-trans-2,3-epoxybutyloxy)-3-methoxyphenolformiat, welches im NMR-Spektrum «Peaks» bei 3,2, 3,6, 3,9, 4,15 und 8,3 ppm zeigt.

Eine Lösung von 8 g der letztgenannten Verbindung in 20 ml Methanol wird unter Rühren und Kühlen mit 35 ml einer 1,75-normalen Kaliumhydroxydlösung versetzt, das Gemisch 18 Stunden bei Zimmertemperatur, gerührt, auf ungefähr sein halbes Volumen konzentriert, mit 50 ml Wasser verdünnt und mit Diäthyläther extrahiert. Der Extrakt wird mit Wasser gewaschen, getrocknet und eingedampft. Man erhält das d,l-erythro-8-Methoxy-2-oxiranyl-1,4benzodioxan als ein Öl, welches im NMR-Spektrum «Peaks» bei 2,8, 3,1, 3,8 und 6,5 ppm aufweist.

*Beispiel 6:*

Ein Gemisch von 1,7 g cis-2,3-bis-Chlormethyloxiran, 1,1 g Brenzcatechin und 2 ml Isopropanol wird auf 65°C erhitzt und mit einer Lösung von 0,8 g Natriumhydroxyd in 2 ml Wasser, unter Rühren, tropfenweise versetzt. Das Reaktionsgemisch wird 60 Minuten auf 85°C erhitzt, abgekühlt und mit Benzol verdünnt. Es wird mit Wasser gewaschen, getrocknet und eingedampft. Man erhält ein Gemisch, welches 48% des als Ausgangsmaterial verwendeten Epoxids, 40% des gewünschten d,l-threo-2-Oxiranylbenzodioxans (Schmelzpunkt 45°C) und 10% 2-Chlormethyl-3-hydroxymethylbenzodioxan enthält (durch Massenspektroskopie bestimmt). Das Gemisch wird durch Kugelrohrdestillation aufgetrennt. Die bei 120°C/0,2 mmHg siedende Fraktion wird aufgefangen. Sie ist die gewünschte threo-Verbindung.

Der Ausgangsstoff wird wie folgt hergestellt: Eine 0,2-molare Lösung von 3-Chlorperbenzoesäure in 275 ml Methylenchlorid wird unter Rühren mit einer Lösung von 25 g cis-1,4-Dichlor-2-buten in 50 ml Methylenchlorid versetzt. Das Gemisch wird bei Zimmertemperatur 4 Tage gerührt, filtriert und das Filtrat 30 Minuten mit 100 ml einer 10%igen wässerigen Natriumsulfitlösung gerührt. Die organische Schicht wird abgetrennt, mit kalter 12%iger wässeriger Natriumhydroxydlösung und Wasser gewaschen, getrocknet und eingedampft. Man erhält das 2,3-bis-Chlormethyloxiran, welches bei 83 bis 85°C/25 mmHg siedet.

*Beispiel 7:*

Eine Lösung von 1,32 g Brenzcatechin in 15 ml Dimethylsulfoxid wird in einer Stickstoffatmosphäre, unter Rühren bei 55°C, mit 0,8 g Natriumhydroxydtabletten versetzt. Nach 4 Stunden wird die dunkelgrüne Flüssigkeit mit 1,5 g trans-2,3-bis-Chlormethyloxiran versetzt und bei 55 bis 60°C 4 Stunden weitergerührt. Nach Abkühlung auf Zimmertemperatur wird das Gemisch mit 100 ml Wasser verdünnt und mit Diäthyläther extrahiert. Der Extrakt wird mit wässeriger Natriumhydroxydlösung und gesättigter wässeriger Natriumchloridlösung gewaschen, getrocknet und eingedampft. Man erhält ein hellgelbes Öl, welches auf Silicagel chromatographiert und mit Chloroform eluiert wird. Nach Eindampfen erhält man ein farbloses Öl, welches nach Stehenlassen fest wird. Das Material wird aus Diäthyläther umkristallisiert. Man erhält das d,l-erythro- oder (R-S) (S-R)-2-Oxiranyl-1,4-benzodioxan der Formel

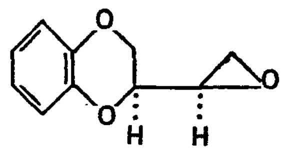

welches bei 51 bis 52°C schmilzt.

Der Ausgangsstoff wird wie folgt hergestellt: Eine Lösung von 0,2 molarer m-Chlorperbenzoesäure in 275 ml Methylenchlorid wird unter Rühren mit einer Lösung von 25 g trans-1,4-Dichlor-2-buten in 50 ml Methylenchlorid versetzt. Das Gemisch wird bei Zimmertemperatur 4 Tage gerührt, filtriert und das Filtrat 30 Minuten mit 100 ml einer 10%igen wässerigen Natriumsulfitlösung gerührt. Die organische Schicht wird abgetrennt, mit kalter 12%iger wässeriger Natriumhydroxydlösung und Wasser gewaschen, getrocknet und eingedampft. Man erhält das trans-2,3-bis-Chlormethyloxiran, welches bei 85 bis 88°C/25 mmHg siedet.

*Beispiel 8:*

Eine Lösung von 1,32 g Brenzcatechin in 60 ml Dimethylformamid wird in einer Stickstoffatmosphäre unter Rühren bei 50 bis 55°C mit 0,96 g einer 50%igen Suspension von Natriumhydrid in Mineralöl versetzt. Nach 30 Minuten wird eine Lösung von 1,5 g cis-2,3-bis-Chlormethyloxiran in 5 ml Dimethylformamid zugegeben und das Gemisch 2½ Stunden weiter erwärmt und gerührt. Das Reaktionsgemisch wird abgekühlt und gemäss Beispiel 7 aufgearbeitet. Man erhält das d,l-threo- oder (R-R) (S-S)-2-Oxiranyl-1,4-benzodioxan der Formel

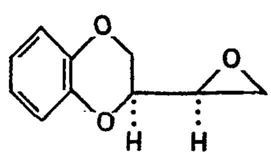

welches bei 45°C schmilzt.

*Beispiel 9:*

Ein Gemisch von 3 g d,l-erythro-2-Oxiranyl-1,4-benzodioxan, 1,9 g 4-Hydroxy-4-phenylpiperidin und 20 ml Isopropanol wird 7 Stunden unter Rückfluss gekocht. Nach Abkühlung auf Zimmertemperatur gibt man unter Rühren 0,7 ml Methansulfonsäure dazu und lässt das Gemisch über Nacht im Kühlschrank stehen. Man erhält das d,l-erythro-2-[2-(4-Hydroxy-4-phenylpiperidino)-1-hydroxyäthyl]-1,4-benzodioxanmethansulfonat,

welches nach Umkristallisation aus wässerigem Isopropanoldiäthyläther bei 212 bis 213°C schmilzt.

Man suspendiert 1 g der erhaltenen Verbindung in 20 ml Wasser, macht das Gemisch mit wässerigem Natriumhydroxyd basisch und extrahiert es mit Methylenchlorid. Der Extrakt wird getrocknet, eingedampft und der Rückstand aus Isopropanol umkristallisiert. Man erhält die freie Base, welche bei 118°C schmilzt. Sie ist identisch mit derjenigen, die im US-Patent 3914238 beschrieben worden ist.

*Beispiel 10:*

Ein Gemisch von 3 g d,l-threo-2-Oxiranyl-1,4-benzodioxan und 20 ml tert.-Butylamin wird in einem Einschmelzrohr 10 Stunden bei 100°C erhitzt und dann eingedampft. Der Rückstand wird in wässerigem Ammoniak aufgenommen, das Gemisch mit Chloroform extrahiert, der Extrakt getrocknet, eingedampft und der Rückstand aus Petroläther umkristallisiert. Man erhält das
d,l-threo-2-(2-tert.-Butylamino-1-hydroxyäthyl)benzodioxan,
welches bei 84 bis 85°C schmilzt.

Die letztgenannte Verbindung wird in einer minimalen Menge Äthanol gelöst, die Lösung mit Chlorwasserstoff in Äthanol angesäuert und mit Diäthyläther verdünnt. Man erhält das entsprechende Hydrochlorid, welches nach Umkristallisation aus Äthanol bei 192 bis 194°C schmilzt. Das Produkt ist mit demjenigen, welches im US-Patent 3312592 beschrieben worden ist, identisch.

Ersetzt man das oben genannte d,l-threo-2-Oxiranyl-1,4-benzodioxan durch die gleiche Menge des erythro-Enantiomeren, so erhält man das d,l-erythro-2-(2-tert.-Butylamino-1-hydroxyäthyl)benzodioxan, welches bei 93 bis 96°C schmilzt. Sein Hydrochlorid schmilzt bei 155 bis 157°C.

*Beispiel 11:*

Ein Gemisch von 2,5 g d,l-erythro-2-Oxiranyl-1,4-benzodioxan, 1,7 g 4-(2-Oxo-1-benzimidazolinyl)piperidin und 15 ml Isopropanol wird 4 Stunden unter Rückfluss gekocht und zu einem kleinen Volumen konzentriert. Das Konzentrat wird mit Benzol verdünnt und mit 5%iger Chlorwasserstoffsäure extrahiert. Man lässt den Extrakt in der Kälte stehen, filtriert den gummiartigen Niederschlag ab und trituriert ihn mit wässerigem Ammoniak und Chloroform. Die organische Lösung wird abgetrennt, getrocknet, eingedampft und der Rückstand aus Essigsäureäthylester umkristallisiert. Man erhält das
d,l-erythro-2-[2(4-(2-Oxo-1-benzimidazolinyl)-piperidino)-1-hydroxyäthyl]-1,4-benzodioxan,
welches bei 188 bis 190°C schmilzt. Das Produkt ist mit demjenigen des deutschen Patents 2400094 identisch.

Die analog erhaltene d,l-threo-Verbindung schmilzt bei 110 bis 115°C.

**Patentansprüche**

1. Stereospezifisches Verfahren zur Herstellung von d,l-erythro- und d,l-threo-2-Oxiranyl-1,4-benzodioxanen der allgemeinen Formel I

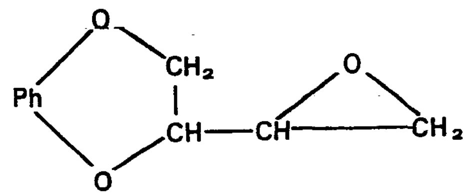

(I)

worin Ph 1,2-Phenylen bedeutet, welches durch ein bis drei gleiche oder verschiedene Substituenten der Gruppe bestehend aus Niederalkyl, Niederalkoxy, Niederalkylendioxy, Benzyloxy, Halogen, Trifluormethyl oder Nitro substituiert sein kann, dadurch gekennzeichnet, dass man in Gegenwart einer starken Base, eine Verbindung der allgemeinen Formel II

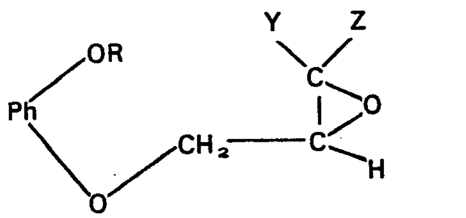

(II)

kondensiert, worin R Formyl oder M beudeutet, wobei M für den Rest einer starken quaternären organischen Stickstoff enthaltenden Base oder für ein Alkalimetallatom $M_a$ steht, eines der Symbole Y und Z Wasserstoff bedeutet und das andere für $X-CH_2$ steht, worin X ein Halogenatom bedeutet, oder, wenn R für M steht, das Symbol X auch eine andere reaktionsfähige veresterte Hydroxygruppe bedeutet, und in welcher Verbindung II die Substituenten des Oxiranringes zueinander in cis-Konfiguration (Z bedeutet Wasserstoff) stehen oder in trans-Konfiguration (Z steht für $CH_2X$) orientiert sind, wobei das zuerst genannte Isomere zu den threo-Verbindungen der Formel I führt, während ein trans-Ausgangsstoff erythro-Verbindungen der Formel I ergibt.

2. Verfahren nach Anspruch 1, dadurch gekennzeichnet, dass man die Kondensation in Gegenwart einer starken Base $M^{\oplus}OH^{\ominus}$, worin M den Rest einer starken quaternären organischen Stickstoff enthaltenden Base oder ein Alkalimetallatom $M_a$ bedeutet.

3. Verfahren nach Anspruch 1, dadurch gekennzeichnet, dass man Ausgangsstoffe verwendet, worin R Formyl bedeutet, und die anderen Symbole die im Anspruch 1 angegebenen Bedeutungen haben.

4. Verfahren nach Anspruch 3, dadurch gekennzeichnet, dass man die Kondensation in Gegenwart eines Alkalimetallhydroxyds oder einer starken quaternären Stickstoff enthaltenden Base durchführt.

5. Verfahren nach Anspruch 3, dadurch gekennzeichnet, dass man als Base eine wässerige Natrium- oder Kaliumhydroxydlösung verwendet.

6. Verfahren nach Anspruch 1, dadurch gekennzeichnet, dass man Ausgangsstoffe verwendet, worin R für M steht, wobei M den Rest einer starken quaternären organischen Stickstoff enthaltenden Base oder ein Alkalimetallatom $M_a$ bedeutet, und die anderen Symbole die im Anspruch 1 angegebenen Bedeutungen haben.

7. Verfahren nach Anspruch 6, dadurch gekennzeichnet, dass man Ausgangsstoffe verwendet, in welchen M Natrium bedeutet, das Symbol X für Chlor steht, und die anderen Symbole die im Anspruch 1 angegebenen Bedeutungen haben.

8. Verfahren nach Anspruch 1, dadurch gekennzeichnet, dass man Ausgangsstoffe verwendet, worin Ph 1,2-Phenylen bedeutet, welches durch Alkyl oder Alkoxy mit jeweils 1-4 Kohlenstoffatomen, Alkylendioxy mit 1 oder 2 Kohlenstoffatomen, Benzyloxy, Fluor, Chlor, Brom, Trifluormethyl oder Nitro monosubstituiert sein kann.

9. Verfahren nach Anspruch 1, dadurch gekennzeichnet, dass man Ausgangsstoffe verwendet, worin Ph 1,2-Phenylen bedeutet, welches durch Methyl, Methoxy, Methylendioxy, Benzyloxy, Fluor, Chlor, Trifluormethyl oder Nitro monosubstituiert sein kann.

10. Verfahren nach einem der Ansprüche 6 oder 7, dadurch gekennzeichnet, dass man Ausgangsstoffe verwendet, worin Ph 1,2-Phenylen bedeutet, welches in 3,6- oder 4,5-Stellungen durch die im Anspruch 1 angegebenen 1-wertigen Substituenten symmetrisch substituiert sein kann.

## Claims

1. A stereospecific process for the manufacture of a d,l-erythro- and d,l-threo-2-oxiranyl-1,4-benzodioxanne of the general formula I:

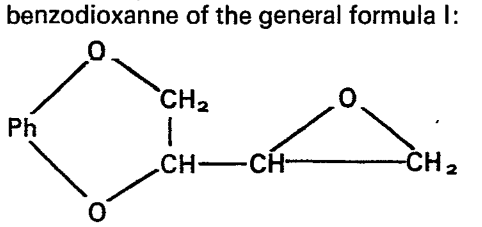 (I)

wherein Ph is 1,2-phenylene which may be substituted by one to three identical or different members selected from lower alkyl, lower alkoxy, lower alkylenedioxy, benzyloxy, halogen, trifluoromethyl or nitro, which process comprises condensing, in the presence of a strong base, a compound of the general formula II:

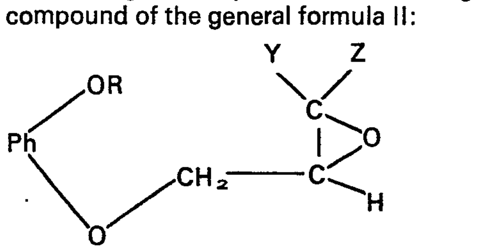 (II)

in which R is formyl or M, and M is the radical of a strong quaternary organic nitrogenous base or an alkali metal atom $M_a$, one of Y and Z is hydrogen and the other is $X-CH_2$, in which X is a halogen atom or, when R is M, X is also an other reactive esterified hydroxyl group, and in which compound II the substituents of the oxirane ring are oriented to each other in the cis-configuration (Z is H) or in the trans-configuration (Z is $CH_2X$), said former isomer resulting in the threo-compound of the formula I, and said latter isomer in the erythro-compound of the formula I.

2. A process according to claim 1, wherein the condensation is carried out in the presence of a strong base $M^{\oplus} OH^{\ominus}$, in which M is the radical of a strong quaternary organic nitrogenous base or an alkali metal atom $M_a$.

3. A process according to claim 1, which comprises the use of starting materials in which R is formyl and the other symbols have the meanings given in claim 1.

4. A process according to claim 3, wherein the condensation is carried out in the presence of an alkali metal hydroxide or a strong quaternary nitrogenous base.

5. A process according to claim 3, wherein an aqueous solution of sodium or potassium hydroxide is used as base.

6. A process according to claim 1, which comprises the use of starting materials in which R is M, and M is the radical of a strong quaternary organic nitrogenous base or an alkali metal atom $M_a$, and the other symbols have the meanings given in claim 1.

7. A process according to claim 6, which comprises the use of starting materials in which M is sodium, X is chlorine, and the other symbols have the meanings given in claim 1.

8. A process according to claim 1, which comprises the use of starting materials in which Ph is 1,2-phenylene, unsubstituted or monosubstituted by alkyl or alkoxy, each of 1 to 4 carbon atoms, alkylenedioxy of 1 or 2 carbon atoms, benzyloxy, fluorine, chlorine or bromine, trifluoromethyl or nitro.

9. A process according to claim 1, which comprises the use of starting materials in which Ph is 1,2-phenylene, unsubstituted or monosubstituted by methyl, methoxy, methylenedioxy, benzyloxy, fluorine, chlorine, trifluoromethyl or nitro.

10. A process according to either of claims 6 or 7, which comprises the use of starting materials in which the 1,2-phenylene ring may be symmetrically substituted in the 3,6- or 4,5-positions by the substituents indicated in claim 1.

## Revendications

1. Procédé stéréospécifique de préparation de d,l-érythro- et d,l-thréo-2-oxiranyl-1,4-benzodioxannes de formule générale I:

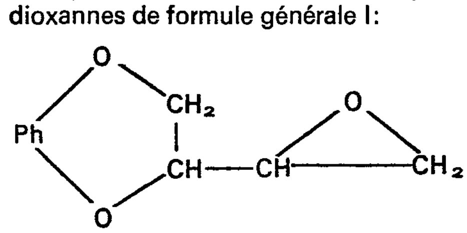 (I)

où Ph représente un 1,2-phénylène, qui peut être substitué par 1 à 3 substituants semblables ou différents du groupe constitué par les alcoyles inférieurs, les alcoxy inférieurs, les alcoylènedioxy inférieurs, le benzyloxy, les halogènes, le trifluorométhyle ou le nitro, caractérisé en ce qu'on condense, en présence d'une base forte, un composé de formule générale II

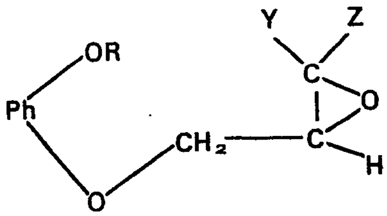

(II)

où R représente un formyle ou M, où M représente le radical d'une base forte contenant un azote organique quaternaire ou un atome de métal alcalin $M_a$, l'un des symboles Y et Z représente un hydrogène et l'autre $X-CH_2$, où X représente un atome d'halogène, ou, lorsque R représente M, le symbole X représente également un autre groupe hydroxy estérifié réactif, composé II dans lequel les substituants du noyau oxirane sont l'un par rapport à l'autre en configuration cis (Z représente un hydrogène) ou en configuration trans (Z représente $CH_2X$), où le premier isomère mentionné aboutit aux composés thréo de formule I, tandis qu'un produit de départ trans conduit aux composés érythro de formule I.

2. Procédé selon la revendication 1, caractérisé en ce qu'on effectue la condensation en présence d'une base forte $M^{\oplus} OH^{\ominus}$, où M représente le radical d'une base forte contenant un azote organique quaternaire ou un atome de métal alcalin $M_a$.

3. Procédé selon la revendication 1, caractérisé en ce qu'on utilise des produits de départ où R représente un formyle et où les autres symboles ont les significations données dans la revendication 1.

4. Procédé selon la revendication 3, caractérisé en ce qu'on effectue la condensation en présence d'un hydroxyde de métal alcalin ou d'une base forte contenant un azote quaternaire.

5. Procédé selon la revendication 3, caractérisé en ce qu'on utilise comme base une solution aqueuse d'hydroxyde de sodium ou de potassium.

6. Procédé selon la revendication 1, caractérisé en ce qu'on utilise des produits de départ où R représente M, M représentant le radical d'une base forte contenant un azote organique quaternaire ou un atome de métal alcalin $M_a$, et où les autres symboles ont les significations données dans la revendication 1.

7. Procédé selon la revendication 6, caractérisé en ce qu'on utilise des produits de départ où M représente le sodium, le symbole X représente un chlore, et où les autres symboles ont les significations données dans la revendication 1.

8. Procédé selon la revendication 1, caractérisé en ce qu'on utilise des produits de départ où Ph représente un 1,2-phénylène qui peut être monosubstitué par un alcoyle ou un alcoxy ayant chacun de 1 à 4 atomes de carbone, un alcoylènedioxy ayant 1 ou 2 atomes de carbone, un benzyloxy, un fluor, un chlore, un brome, un trifluorométhyle ou un nitro.

9. Procédé selon la revendication 1, caractérisé en ce qu'on utilise des produits de départ où Ph représente un 1,2-phénylène qui peut être monosubstitué par un méthyle, un méthoxy, un méthylènedioxy, un benzyloxy, un fluor, un chlore, un trifluorométhyle ou un nitro.

10. Procédé selon l'une des revendications 6 ou 7, caractérisé en ce qu'on utilise des produits de départ où Ph représente un 1,2-phénylène qui peut être substitué de façon symétrique dans les positions 3,6 ou 4,5 par les substituants monovalents énumérés dans la revendication 1.